# EUROPEAN PATENT SPECIFICATION

(11) **EP 3 478 264 B1**
(45) Date of publication and mention of the grant of the patent: **19.11.2025**
(21) Application number: 17732583.4
(22) Date of filing: 14.06.2017
(51) Int. Cl.: A61K 9/00, A61L 9/16, A61K 9/16, A61P 25/34, A61K 31/465

(54) **NICOTINE PARTICLES AND COMPOSITIONS**
NIKOTINPARTIKEL UND ZUSAMMENSETZUNGEN
PARTICULES ET COMPOSITIONS DE NICOTINE

(30) Priority: 30.06.2016 EP 16177163
(43) Date of publication of application: 08.05.2019
(73) Proprietor: Philip Morris Products S.A., 2000 Neuchâtel (CH)
(72) Inventor: ZUBER, Gerard, 1063 Boulens (CH); VOLPE, Nicolo, 1012 Lausanne (CH)
(74) Representative: Reddie & Grose LLP
(86) International application number: PCT/IB2017/053543
(87) International publication number: WO 2018/002756

(56) References cited:
- WO-A1-99/45902
- WO-A2-01/13893
- US-A1- 2004 118 007
- US-A1- 2015 283 070
- REINHARD VEHRING: "Pharmaceutical Particle Engineering via Spray Drying", PHARMACEUTICAL RESEARCH, KLUWER ACADEMIC PUBLISHERS-PLENUM PUBLISHERS, NL, vol. 25, no. 5, 28 November 2007 (2007-11-28), pages 999 - 1022, XP019613056, ISSN: 1573-904X

## Description

This disclosure relates to nicotine particles and compositions that are suitable for inhalation. These nicotine particles and compositions include nicotine, a sugar, and an amino acid or short peptide.

Dry powder inhalers (DPI) are known and are used to treat respiratory diseases by delivering a dry powder comprising a pharmaceutically active compound, in aerosol form through inhalation to the patients' airways. In pharmaceutical dry powders, the active pharmaceutical ingredient (API) is usually agglomerated on the surface of larger carrier particles, such as lactose for example. DPI's operate complex mechanisms to ensure such agglomerates disperse, break up or disaggregate before the API is inhaled into the lungs.

It may be difficult to deliver nicotine particles to the lungs at inhalation at air flow rates that are within conventional smoking regime inhalation or air flow rates. Nicotine particles may have a tendency to agglomerate and stick to inhaler or processing surfaces, especially as a size of the nicotine particle deceases. Nicotine particles with an MMAD of less than about 10 micrometres tend to be increasingly thermodynamically unstable due to a high surface area to volume ratio, which provides an increasing surface free energy with this decreasing particle size, and consequently increases the tendency of particles to agglomerate and the strength of the agglomerate. Forming nicotine particles may be difficult and costly.

US 2015/283070 relates to a dry powder nicotine formulation suitable for inhalation. The formulation includes nicotine particles, wherein the nicotine particles are substantially in the range of about 1-10 micron in size, preferably 2-5 micron in size. The formulation may also include a first cough suppressant component having particles in the 5-100 micron size range. The formulation may also include a second cough suppressant component having particles in the 10-200 micron size range. The formulation may also include a flavor component having particles in the 10-1000 micron size range.

WO 01/13893 relates to particles having a tap density of less than 0.4 g/cm³ including a hydrophobic amino acid or salt thereof and a therapeutic, prophylactic or diagnostic agent or any combination thereof. The particles may be formed by spray-drying and are useful for delivery to the pulmonary system.

It would be desirable to provide nicotine particles and compositions that may be formed and processed easily. It would be desirable that the nicotine particles and compositions not stick to processing surfaces or agglomerate and exhibit a stable particle size distribution. It would also be desirable that the nicotine particles and compositions be deliverable to the lungs at air flow rates that are within conventional smoking regime inhalation or air flow rates.

The invention resides in a particle that comprises nicotine, a sugar and leucine. The particle preferably has a size in a range from about 0.5 to about 10 micrometres, or from about 0.5 to about 5 micrometres, as measured by a cascade impactor. The particle preferably comprises about 25 wt% or less nicotine or from about 5 to about 15 wt% nicotine. A free flowing composition may be formed by these particles.

The invention also resides in a method of forming the particles. The particles may be formed by combining nicotine, a sugar, and leucine in a liquid carrier to form a liquid mixture. This liquid mixture is spray dried to form a plurality of particles having a size in a range from about 0.5 to about 10 micrometres or in a range from about 0.5 to about 5 micrometres. The plurality of particles is preferably homogenous particles.

Advantageously, the nicotine particles and powder formulation described herein provide for a homogenous and stable particle size sufficient to deliver nicotine to the lungs of a consumer at inhalation or air flow rates that are within conventional smoking regime inhalation or air flow rates. The nicotine particles and powder formulation described herein allows these particles to be formed by spray drying to achieve a specific and controlled particle size distribution while minimizing agglomeration or adherence to surfaces such as processing equipment surfaces. Spray drying may provide a scalable, precise and low cost particle formation unit operation.

The term "nicotine" refers to nicotine and nicotine derivatives in any form, including but not limited to, a free-base nicotine, nicotine salt, or in a matrix such as a sugar matrix or organometallic complex.

The term "amino acid" refers to a single unmodified or modified amino acid moiety, preferably unmodified.

The term "short peptide" refers to a peptide comprising two or three amino acids.

The size of a particle, as stated herein, preferably refers to the aerodynamic diameter of the particle. The aerodynamic diameter of a powder system is preferably measured with a cascade impactor. The term "MMAD" refers to the mass median aerodynamic diameter.

This disclosure relates to particles comprising nicotine, a sugar, and leucine. Particles may be formed having a specific particle size distribution. In illustrative examples, about 90%, or about 95%, or about 98% of the particles have a size of about 5 micrometres or less, or about 4.5 micrometres or less, or about 4.2 micrometres or less, and about 50% of the particles have a size of about 2.5 micrometres or less, or about 2.1 micrometres or less. In many of these examples, about 10% of the particles have a size of about 820 nanometers or less. The particles may have a mass median aerodynamic diameter in a range from about 1 to about 4 micrometres. Substantially all of the particles may have a particle size in a range from about 500 nanometers to about 5 micrometres.

Compositions of these particles have a specific particle size distribution. In illustrative examples, about 90%, or about 95%, or about 98% of the particles of the composition have a size of about 5 micrometres or less, or about 4.5 micrometres or less, or about 4.2 micrometres or less, and about 50% of the particles have a size of about 2.5 micrometres or less, or about 2.1 micrometres or less. In many of these examples, about 10% of the particles have a size of about 820 nanometers or less. The particles of the composition may have a mass median aerodynamic diameter in a range from about 1 to about 4 micrometres. Substantially all of the particles forming the composition may have a particle size in a range from about 500 nanometers to about 5 micrometres. The percentages relating to particle size distribution described herein are based on particles by volume (% by volume).

The nicotine component of the particle may be a free base nicotine, a nicotine salt, or a combination thereof. The nicotine component may be a nicotine salt formed by combining nicotine or nicotine free base with an acid. The acid may be a stoichiometric amount of acid to the nicotine free base, or a stoichiometric excess of acid may be combined with the nicotine free base, or a stoichiometric excess of nicotine free base may be combined with the acid. A free base nicotine may be utilized without the addition of an acid.

The acid may be an organic acid, an inorganic acid, or a Lewis acid. Non-limiting examples of inorganic acids are hydrochloric, hydrobromic, hydroiodic, nitric, sulfuric, phosphoric, acetic, hexafluorophosphoric, and the like. Non-limiting examples of organic acids are levulinic, citric, gluconic, benzoic, propionic, butyric, sulfosalicylic, maleic, lauric, malic, fumaric, succinic, tartaric, amsonic, pamoic, mesylic, aspartic, formic, acetic, propionic, succinic, camphorsulfonic, fumaric, isethionic, lactic, mucic, para-toluenesulfonic, glycolic, glucuronic, maleic, furoic, glutamic, benzoic, anthranilic, salicylic, phenylacetic, pyruvic, mandelic, embonic (pamoic), methanesulfonic, ethanesulfonic, pantothenic, benzenesulfonic (besylate), stearic, sulfanilic, alginic, galacturonic, and the like. Non-limiting examples of Lewis acids are zinc chloride or zinc bromide (ZnCl₂ / ZnBr₂). These can react with nicotine to form organometallic complexes.

Useful nicotine salts include, but are not limited to, nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. Preferred nicotine salts include, nicotine lactate, nicotine pyruvate, nicotine citrate, nicotine aspartate, or a combination thereof.

The pH of the particles (dissolved in water) may be in a range from about 5 to about 9. Preferably the pH is about 7.0 or higher or in a range from 7.0 to 9.0. A pH of 9 can be reached for a particle without organic acid, while a pH of 5.0 can be obtained with the use of a strong acid or diacid when forming the nicotine salt.

The particles include leucine or a leucine isomer such as, L-leucine. Leucine or a leucine isomer may reduce adhesion forces of the particles forming the composition and mitigate or prevent agglomeration of the particles forming the composition. The particles forming the composition described herein thus may be a free flowing material and possess a stable relative particle size distribution during processing, transport and storage.

The particles may further comprise a short peptide (formed of three or fewer amino acids). The short peptide may comprise trileucine, for example.

The particle includes a sugar. Sugar refers to simple sugars, monosaccharides, disaccharides, and polysaccharides. Without limitation, examples of suitable sugars are lactose, sucrose, raffinose, trehalose, fructose, dextrose, glucose, maltose, mannitol, or combinations thereof. Preferred sugars include trehalose or mannitol.

The particle may contain less than about 30 wt% nicotine. The particle may contain about 25 wt% or less nicotine, or from about 15 to about 25 wt% nicotine. The particle may contain from about 1 to about 20 wt% nicotine, or from about 10 to about 20 wt% nicotine, or from about 5 to 15 wt% nicotine. The particle may contain from about 1 to about 10 wt% nicotine or from about 5 to about 10 wt% nicotine. In some embodiments, particles that contained about 30 wt% or more nicotine agglomerated or adhered to processing surfaces when processed through a spray dryer.

The particles forming the composition may contain less than about 30 wt% nicotine. The particles forming the composition may contain about 25 wt% or less nicotine, or from about 15 to about 25 wt% nicotine. The particles forming the composition may contain from about 1 to about 20 wt% nicotine, or from about 10 to about 20 wt% nicotine, or from about 5 to 15 wt% nicotine. The particles forming the composition may contain from about 1 to about 10 wt% nicotine or from about 5 to about 10 wt% nicotine. In some embodiments, particles forming the composition that contained about 30 wt% or more nicotine produced an agglomerated or sticky composition when processed through a spray dryer.

The particle may contain about 1 to about 10 wt% leucine. The particle may contain about 3 to about 7 wt% leucine. The particle may contain from about 5 wt% leucine. The addition of the leucine, especially L-leucine for example, to the particles may reduce agglomeration or adherence to processing surfaces.

The particles forming the composition may contain about 1 to about 10 wt% leucine. The particles forming the composition may contain about 3 to about 7 wt% leucine. The particles forming the composition may contain from about 5 wt% leucine. The addition of the leucine, especially L-leucine for example, to the particles forming the composition may reduce agglomeration or stickiness of the composition when processed through a spray dryer.

The particle may contain about 60 to about 95 wt% sugar. The particle may contain about 70 to about 90 wt% sugar. The particle may contain about 80 to about 85 wt% sugar.

The particles forming the composition may contain about 60 to about 95 wt% sugar. The particles forming the composition may contain about 70 to about 90 wt% sugar. The particles forming the composition may contain about 80 to about 85 wt% sugar.

A useful particle formulation includes leucine, a sugar being trehalose, and a nicotine salt being nicotine lactate. The nicotine content may be from about 5 to about 15 wt% or about 9.5 wt%. The leucine content may be from about 1 to about 10 wt% The leucine content may be from about 3 to about 7 wt% or about 5 wt%. The molar ratio of acid:nicotine may about 1:1.

A useful particle formulation includes leucine, a sugar being trehalose, and a nicotine salt being nicotine citrate. The nicotine content may be from about 5 to about 15 wt% or about 9.6 wt%. The leucine content may be from about 1 to about 10 wt% The leucine content may be from about 3 to about 7 wt% or about 5 wt%. The molar ratio of acid:nicotine may about 0.25:1.

A useful particle formulation includes leucine, a sugar being trehalose, and a nicotine salt being nicotine pyruvate. The nicotine content may be from about 5 to about 15 wt%or about 9.8 wt%. The leucine content may be from about 1 to about 10 wt% The leucine content may be from about 3 to about 7 wt% or about 5 wt%. The molar ratio of acid:nicotine may about 0.6:1.

A useful particle formulation includes leucine, a sugar being trehalose, and a nicotine salt being nicotine aspartate. The nicotine content may be from about 5 to about 15 wt% or about 9.3 wt%. The leucine content may be from about 1 to about 10 wt% The leucine content may be from about 3 to about 7 wt% or about 5 wt%. The molar ratio of acid:nicotine may about 0.6:1.

The particles may be formed by: (1) combining a nicotine, a sugar, and leucine in a liquid carrier to form a liquid mixture; and (2) spray drying the liquid mixture to form particles having a size in a range from about 0.5 to about 10 micrometres or in a range from about 0.5 to about 5 micrometres.

An illustrative example comprises a preparation that includes a 20% nicotine free base and an acid (e.g., lactic, pyruvic or citric) combined in a liquid carrier. The molar ratio may be within the ranges 1.00:1.20 for nicotine: aspartic, pyruvic or lactic acid, and 0.33:0.50 for nicotine:citric acid. The liquid mixture may be incubated at about 30°C, for example, for about 1 to about 15 minutes, to allow the formation of a stable nicotine salt solution. A pharmaceutically acceptable sugar, (for example, trehalose or mannitol) and leucine may be added to form a liquid mixture. The liquid mixture may be spray dried by using a nozzle to atomize the liquid to form droplets, contacting the droplets with warm air, to dry and form dry particles, and collecting the particles. In this embodiment, after spray drying, 10% of the particles (by volume) may be below about 0.82 micrometre in size, 50% of the particles may be below about 2.1 micrometres in size and 90% of the particles may be below about 4.1 micrometres in size. The particles are substantially in the range of 0.5 to 4.2 micrometres.

The liquid carrier may be water, for example. The liquid mixture is flowable. The liquid mixture is configured to flow through an atomization or atomizer nozzle to form the precise or controlled particle size distribution. The particles or composition may be processed by spray drying to form a precise size distribution of particles. The particles and compositions described herein may tend to not agglomerate or stick to the surface of the spray drying equipment.

The particles and compositions described herein may be processed at a reduced temperature (as compared to conventional nicotine particle formation) resulting in a reduced product loss. For example, the particles and composition described herein may be spray dried at a temperature in a range from about 50 to 85 degrees Celsius.

A cough suppressant may be combined with the composition. Cough suppressants include, for example, menthol, camphor, turpentine oil (e.g., alpha-pinene, beta-pinene) and menthol derivatives (e.g., menthyl lactate, and menthyl salicylate).

The particles and compositions described herein may then be packaged for consumption. The particles and compositions described herein may be packaged in an inhalation delivery consumable element or contained within an inhalation delivery consumable element. An inhalation delivery consumable element may be a capsule, for example. The capsule may be by disposed in an inhalation device, such as a dry powder inhaler. The inhalation device may pierce the capsule and the fine particles may be entrained in the inhalation air for delivery to the lungs of a consumer.

The particles and compositions described herein and the inhalation delivery consumable element may be free of, or substantially free of carrier particles. The particles and compositions described herein and the inhalation delivery consumable element may be free of, or substantially free of particles that are greater than about 20 micrometres, or greater than about 50 micrometres, or greater than about 100 micrometres.

The nicotine may be dissolved in the liquid carrier to form the liquid mixture. The sugar may be dissolved in the liquid carrier to form the liquid mixture. The amino acid may be dissolved in the liquid carrier to form the liquid mixture. The liquid mixture may have about 20% w/v or less total solids, or about 15% w/v or less total solids, or a range of about 5 to 15% w/v total solids.

The nicotine particles described herein may be processed at a reduced (as compared to conventional nicotine particles) temperature that may result in reduced product loss. The spray drying inlet temperature and the outlet temperature may be reduced. The spray drying atomization pressure may be in a range from about 3 to about 7 bar, or 4 to about 6 bar, or about 5 bar.

The spray drying inlet temperature may be about 140 degrees Celsius or less, or about 135 degrees Celsius or less, or about 130 degrees Celsius or less, or in a range from about 100 to about 1500 degrees Celsius, or in a range from about 110 to about 140 degrees Celsius, or in a range from about 125 to about 135 degrees Celsius. The spray drying outlet temperature may be about 100 degrees Celsius or less, or about 95 degrees Celsius or less, or about 90 degrees Celsius or less, about 85 degrees Celsius or less, or about 80 degrees Celsius or less, or in a range from about 30 to about 90 degrees Celsius, or in a range from about 40 to about 90 degrees Celsius, or in a range from about 50 to about 85 degrees Celsius.

Specific examples are set forth in the tables below.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

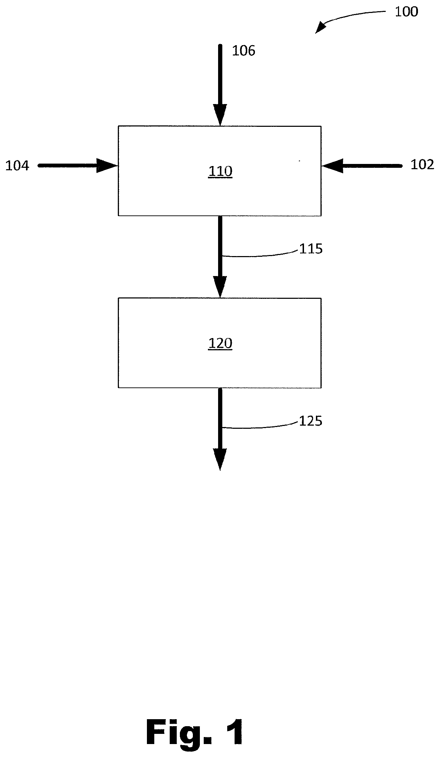

**FIG.** 1 is a schematic flow diagram of an illustrative method **100** of forming the particles **125.** The method **100** includes combining nicotine **102,** a sugar **104,** and an amino acid or peptide **106** in a liquid carrier to form a liquid mixture 115 at block 110. Then, at block 120, the liquid mixture 115 is spray dried to form a plurality of particles 125.

### Examples

All the examples (except **Table** 3 examples) are formulated by combining a nicotine free base and an acid in water (at the specified ratio) to form a stable nicotine salt solution. Then the sugar and amino acid (leucine) is combined with the nicotine salt solution to form a liquid mixture. Then the liquid mixture is atomized and dried to form dry particles that are collected to from the composition.

The **Table** 3 examples are formulated by combining a nicotine free base with sugar and an amino acid (leucine) to form a liquid mixture. Then the liquid mixture is atomized and dried to form dry particles that are collected to from the composition.

The spray dryer was a Buchi B-290 spray dryer (available from Buchi Corp., DE, USA). The liquid mixture was provided to the spray dryer at a flow rate of 2 ml/min at 5 bar atomization pressure. The outlet temperature was about 80 degrees Celsius for examples utilizing trehalose. **Table a1** below describes lactic acid formulations. **Table 2** below describes pyruvic acid formulations. **Table 3** below describes no acid formulations. **Table 4** and **Table 5** report the particle size distribution of various examples.

**Table 1 - Lactic Acid Nicotine Powder Formulations**

| **Example** | **Formulation** | **pH of powder solution** | **Comments** |
|---|---|---|---|
| **L1** | 10% Nicotine, Lactic acid (1:1), 85% Trehalose | 7.3 | Small amount of powder adhering to spray dryer surface |
| **L2** | 15% Nicotine, Lactic acid (1:1), 77% Trehalose | 7.0 | Small amount of powder adhering to spray dryer surface |
| **L3** | 10% Nicotine, Lactic acid (1:1), 80% Trehalose, 5% Leucine | 7.5 | Free flowing powder - no adherence |
| **L4** | 15% Nicotine, Lactic acid (1:1), 72% Trehalose, 5% Leucine | 7.1 | Free flowing powder - no adherence |
| **L5** | 20% Nicotine, Lactic acid (1:1), 64% Trehalose, 5% Leucine | -- | Free flowing powder - no adherence |

**Table 2 - Pyruvic Acid Nicotine Powder Formulations**

| **Example** | **Formulation** | **pH of powder solution** | **Comments** |
|---|---|---|---|
| **P1** | 10% Nicotine, Pyruvic acid (0.6:1), 87% Trehalose | 7.5 | Powder adhering to spray dryer surface, cohesive powder |
| **P2** | 15% Nicotine, Pyruvic acid (0.6:1), 80% Trehalose | 7.8 | Cohesive powder, some static charge |
| **P3** | 10% Nicotine, Pyruvic acid (0.6:1), 82% Trehalose, 5% Leucine | 7.7 | Free flowing powder - no adherence, some static charge |
| **P4** | 15% Nicotine, Pyruvic acid (0.6:1), 75% Trehalose, 5% Leucine | 7.8 | Free flowing powder - no adherence |
| **P5** | 20% Nicotine, Pyruvic acid (0.6:1), 68% Trehalose, 5% Leucine | 7.7 | Free flowing powder - no adherence |

**Table 3 - No Acid (Free Base) Nicotine Powder Formulations**

| **Example** | **Formulation** | **pH of powder solution** | **Comments** |
|---|---|---|---|
| **N1** | 10% Nicotine, 90% Trehalose | 9.3 | Some powder adhering to spray dryer surface |
| **N2** | 15% Nicotine, 85% Trehalose | 9.5 | Some powder adhering to spray dryer surface |
| **N3** | 10% Nicotine, 85% Trehalose, 5% Leucine | 8.6 | Free flowing powder - no adherence, some static charge |
| **N4** | 15% Nicotine, 80% Trehalose, 5% Leucine | 8.7 | Free flowing powder - no adherence |
| **N5** | 20% Nicotine, 75% Trehalose, 5% Leucine | 8.8 | Free flowing powder - no adherence |

**Table 4 - Particle Size Distribution - reported in micrometres**

| **Example** | **X₁₀** | **X₅₀** | **X₉₀** | **VMD** |
|---|---|---|---|---|
| **L1** | 0.65 | 1.43 | 3.54 | 1.81 |
| **L2** | 0.68 | 1.62 | 3.75 | 1.97 |
| **L3** | 0.76 | 1.89 | 3.86 | 2.14 |
| **L4** | 0.92 | 2.14 | 3.99 | 2.35 |
| **L5** | 0.78 | 1.95 | 3.90 | 2.19 |
| **P1** | 0.67 | 1.54 | 3.47 | 1.85 |
| **P2** | 0.67 | 1.53 | 3.54 | 1.86 |
| **P3** | 0.66 | 1.48 | 3.54 | 1.84 |
| **P4** | 0.72 | 1.78 | 3.79 | 2.06 |
| **P4** | 0.65 | 1.43 | 3.54 | 1.81 |
| **N1** | 0.68 | 1.62 | 3.75 | 1.97 |
| **N2** | 0.76 | 1.89 | 3.86 | 2.14 |
| **N3** | 0.92 | 2.14 | 3.99 | 2.35 |
| **N4** | 0.78 | 1.95 | 3.90 | 2.19 |
| **N5** | 0.67 | 1.54 | 3.47 | 1.85 |

| | | | | |
|---|---|---|---|---|
| X₁₀ refers to size of particle where 10% of particles, by volume, are less than this size. X₅₀ refers to size of particle where 50% of particles, by volume, are less than this size. X₉₀ refers to size of particle where 90% of particles, by volume, are less than this size. VMD refers to volume mean diameter. | | | | |

**Table 5 - Further Formulations**

| **Example** | **Formulation** | **X₁₀** | **X₅₀** | **X₉₀** | **VMD** | **MMAD** |
|---|---|---|---|---|---|---|
| **1** | 10% Nicotine, Lactic Acid (1:1), 80% Trehalose, 5% Leucine | 0.92 | 2.17 | 4.15 | 2.4 | 3.8 |
| **2** | 10% Nicotine, Pyruvic Acid (1:0.6), 82% Trehalose, 5% Leucine | 1.04 | 2.56 | 5.08 | 2.9 | 4.0 |
| **3** | 10% Nicotine, Citric Acid (1:0.25), 82% Trehalose, 5% Leucine | 0.81 | 2.34 | 5.48 | 2.8 | 3.5 |
| **4** | 10% Nicotine, Aspartic Acid (1:0.6), 80% Trehalose, 5% Leucine | 0.82 | 2.24 | 4.96 | 2.6 | 4.2 |
| **5** | 5% Nicotine, Lactic Acid (1:1), 82% Trehalose, 10% Leucine | 0.7 | 1.5 | 3.0 | 1.5 | 2.5 |

## Claims

1. A particle (125), comprising:
nicotine (102);
a sugar (104); and
leucine (106).

2. The particle (125) of claim 1 wherein the particle has a size in a range from about 0.5 to about 10 micrometres or in a range from about 0.5 to about 5 micrometres, as measured by a cascade impactor.

3. The particle (125) of any one of the preceding claims, wherein the sugar comprises trehalose, or mannitol.

4. The particle (125) of any one of the preceding claims, wherein the nicotine comprises a nicotine salt selected from the group consisting of nicotine lactate, nicotine pyruvate, nicotine citrate, and nicotine aspartate.

5. The particle (125) of any one of the preceding claims, wherein the sugar comprises trehalose and the nicotine comprises nicotine lactate.

6. The particle (125) of any one of claims 1 to 4, wherein the nicotine comprises nicotine citrate or nicotine aspartate.

7. The particle (125) of any one of the preceding claims, wherein the particle comprises about 25 wt% or less nicotine or from about 5 to about 15 wt% nicotine.

8. The particle (125) of any one of the preceding claims, wherein the particle comprises about 60 wt% to about 95 wt% sugar or from about 70 to about 90 wt% sugar.

9. The particle (125) of any one of the preceding claims, wherein the particle comprises about 1 wt% to about 10 wt% leucine or from about 3 to about 7 wt% leucine.

10. A composition comprising a plurality of particles (125) according to any one of the preceding claims, wherein about 90% of the plurality of particles have a particle size of about 4.5 micrometres or less, and about 50% of the plurality of particles have a particle size of less than about 2.5 micrometers, as measured by cascade impactor.

11. A method (100), comprising:
combining nicotine (102), a sugar (104), and leucine (106) in a liquid carrier to form a liquid mixture (115); and
spray drying the liquid mixture to form a plurality of particles having a size in a range from about 0.5 to about 10 micrometres or in a range from about 0.5 to about 5 micrometres.

12. The method (100) according to claim 11, wherein the combining step comprises combining a cough suppressant with the nicotine, sugar, and leucine in the liquid carrier to form the liquid mixture.

13. The method (100) according to claim 10 or 11, wherein the nicotine is a nicotine salt selected from the group consisting of nicotine lactate, nicotine pyruvate, nicotine citrate, and nicotine aspartate, and the sugar comprises trehalose, mannitol, sucrose, or lactose.

14. The method (100) according to any one of claims 11 to 13, wherein the sugar comprises trehalose, and the dry powder composition comprises about 25 wt% or less nicotine or from about 5 to about 15 wt% nicotine.

## Patentansprüche

1. Partikel (125), aufweisend:
Nikotin (102);
einen Zucker (104); und
Leucin (106).

2. Partikel (125) nach Anspruch 1, wobei das Partikel eine Größe in einem Bereich von etwa 0,5 bis etwa 10 Mikrometer oder in einem Bereich von etwa 0,5 bis etwa 5 Mikrometer aufweist, wie mit einem Kaskadenimpaktor gemessen.

3. Partikel (125) nach einem der vorhergehenden Ansprüche, wobei der Zucker Trehalose oder Mannitol aufweist.

4. Partikel (125) nach einem der vorhergehenden Ansprüche, wobei das Nikotin ein Nikotinsalz aufweist, ausgewählt aus der Gruppe bestehend aus Nikotinlactat, Nikotinpyruvat, Nikotincitrat und Nikotinaspartat.

5. Partikel (125) nach einem beliebigen der vorhergehenden Ansprüche, wobei der Zucker Trehalose aufweist und das Nikotin Nikotinlactat aufweist.

6. Partikel (125) nach einem der Ansprüche 1 bis 4, wobei das Nikotin Nikotincitrat oder Nikotinaspartat aufweist.

7. Partikel (125) nach einem beliebigen der vorhergehenden Ansprüche, wobei das Partikel etwa 25 Gew.-% oder weniger Nikotin oder von etwa 5 bis etwa 15 Gew.-% Nikotin aufweist.

8. Partikel (125) nach einem beliebigen der vorhergehenden Ansprüche, wobei das Partikel etwa 60 Gew.-% bis etwa 95 Gew.-% Zucker oder von etwa 70 bis etwa 90 Gew.-% Zucker aufweist.

9. Partikel (125) nach einem beliebigen der vorhergehenden Ansprüche, wobei das Partikel etwa 1 Gew.-% bis etwa 10 Gew.-% Leucin oder von etwa 3 bis etwa 7 Gew.-% Leucin aufweist.

10. Zusammensetzung, umfassend eine Mehrzahl von Partikeln (125) nach einem beliebigen der vorhergehenden Ansprüche, wobei etwa 90 % der Mehrzahl von Partikeln eine Partikelgröße von etwa 4,5 Mikrometer oder weniger aufweisen und etwa 50 % der Mehrzahl von Partikeln eine Partikelgröße von weniger als etwa 2,5 Mikrometer aufweisen, wie gemessen mit einem Kaskadenimpaktor.

11. Verfahren (100), umfassend:
Kombinieren von Nikotin (102), einem Zucker (104) und Leucin (106) in einem flüssigen Träger, um ein flüssiges Gemisch (115) zu bilden; und
Sprühtrocknen des flüssigen Gemisches, um eine Mehrzahl von Partikeln zu bilden, die eine Größe in einem Bereich von etwa 0,5 bis etwa 10 Mikrometer oder in einem Bereich von etwa 0,5 bis etwa 5 Mikrometer aufweisen.

12. Verfahren (100) nach Anspruch 11, wobei der Kombinationsschritt ein Kombinieren eines Hustenreizdämpfers mit dem Nikotin, dem Zucker und Leucin in dem flüssigen Träger umfasst, um das flüssige Gemisch zu bilden.

13. Verfahren (100) nach Anspruch 10 oder 11, wobei das Nikotin ein Nikotinsalz ist, ausgewählt aus der Gruppe bestehend aus Nikotinlactat, Nikotinpyruvat, Nikotincitrat und Nikotinaspartat, und der Zucker Trehalose, Mannitol, Saccharose oder Lactose aufweist.

14. Verfahren (100) nach einem beliebigen der Ansprüche 11 bis 13, wobei der Zucker Trehalose aufweist und die Trockenpulverzusammensetzung etwa 25 Gew.-% oder weniger Nikotin oder etwa 5 bis etwa 15 Gew.-% Nikotin aufweist.

## Revendications

1. Particule (125), comprenant :
nicotine (102) ;
un sucre (104) ; et
leucine (106).

2. Particule (125) selon la revendication 1, dans laquelle la particule a une taille dans une plage d'environ 0,5 à environ 10 micromètres ou dans une plage d'environ 0,5 à environ 5 micromètres, telle que mesurée par un impacteur en cascade.

3. Particule (125) selon l'une quelconque des revendications précédentes, dans laquelle le sucre comprend du tréhalose ou du mannitol.

4. Particule (125) selon l'une quelconque des revendications précédentes, dans laquelle la nicotine comprend un sel de nicotine choisi dans le groupe comprenant lactate de nicotine, pyruvate de nicotine, citrate de nicotine et aspartate de nicotine.

5. Particule (125) selon l'une quelconque des revendications précédentes, dans laquelle le sucre comprend du tréhalose et la nicotine comprend du lactate de nicotine.

6. Particule (125) selon l'une quelconque des revendications 1 à 4, dans laquelle la nicotine comprend du citrate de nicotine ou de l'aspartate de nicotine.

7. Particule (125) selon l'une quelconque des revendications précédentes, dans laquelle la particule comprend environ 25 % en poids ou moins de nicotine ou environ 5 à environ 15 % en poids de nicotine.

8. Particule (125) selon l'une quelconque des revendications précédentes, dans laquelle la particule comprend environ 60 % en poids à environ 95 % en poids de sucre ou environ 70 à environ 90 % en poids de sucre.

9. Particule (125) selon l'une quelconque des revendications précédentes, dans laquelle la particule comprend environ 1 % en poids à environ 10 % en poids de leucine ou environ 3 à environ 7 % en poids de leucine.

10. Composition comprenant une pluralité de particules (125) selon l'une quelconque des revendications précédentes, dans laquelle environ 90 % de la pluralité de particules ont une taille de particule d'environ 4,5 micromètres ou moins, et environ 50 % de la pluralité de particules ont une taille de particule inférieure à environ 2,5 micromètres, telle que mesurée par un impacteur en cascade.

11. Procédé (100), comprenant :
la combinaison de nicotine (102), d'un sucre (104) et de leucine (106) dans un véhicule liquide pour former un mélange liquide (115) ; et
le séchage par pulvérisation du mélange liquide pour former une pluralité de particules ayant une taille dans une plage d'environ 0,5 à environ 10 micromètres ou dans une plage d'environ 0,5 à environ 5 micromètres.

12. Procédé (100) selon la revendication 11, dans lequel l'étape de combinaison comprend la combinaison d'un antitussif avec la nicotine, le sucre et la leucine dans le véhicule liquide pour former le mélange liquide.

13. Procédé (100) selon la revendication 10 ou 11, dans lequel la nicotine est un sel de nicotine choisi dans le groupe comprenant lactate de nicotine, pyruvate de nicotine, citrate de nicotine et aspartate de nicotine, et le sucre comprend le tréhalose, le mannitol, le saccharose ou le lactose.

14. Procédé (100) selon l'une quelconque des revendications 11 à 13, dans lequel le sucre comprend du tréhalose, et la composition de poudre sèche comprend environ 25 % en poids ou moins de nicotine ou environ 5 à environ 15 % en poids de nicotine.
